(19) Europäisches Patentamt
European Patent Office
Office européen des brevets

(11) Veröffentlichungsnummer: **0 029 945**
**B1**

(12) # EUROPÄISCHE PATENTSCHRIFT

(45) Veröffentlichungstag der Patentschrift: **15.09.82**

(51) Int. Cl.$^3$: **C 07 C 175/00**, C 07 C 47/21, C 07 C 11/21, H 01 B 1/12, H 01 L 31/06, C 08 K 5/01

(21) Anmeldenummer: **80107050.9**

(22) Anmeldetag: **14.11.80**

(54) **Verfahren zur Herstellung elektrisch leitfähiger Polyene und deren Verwendung in der Elektrotechnik und zur antistatischen Ausrüstung von Kunststoffen.**

(30) Priorität: **28.11.79 DE 2947797**

(43) Veröffentlichungstag der Anmeldung: **10.06.81 Patentblatt 81/23**

(45) Bekanntmachung des Hinweises auf die Patenterteilung: **15.09.82 Patentblatt 82/37**

(84) Benannte Vertragsstaaten: **BE CH DE FR IT LI NL**

(56) Entgegenhaltungen:
**EP-A-0 000 829**
**GB-A-1 535 367**
**US-A-3 057 947**

(73) Patentinhaber: **BASF Aktiengesellschaft, Carl-Bosch-Strasse 38, D-6700 Ludwigshafen (DE)**

(72) Erfinder: **Feichtmayr, Franz, Dr., Mundenheimer Strasse 158, D-6700 Ludwigshafen (DE)**
Erfinder: **Naarmann, Herbert, Dr., Haardtblick 15, D-6719 Wattenheim (DE)**
Erfinder: **Paust, Joachim, Dr., Ringstrasse 3, D-6701 Neuhofen (DE)**
Erfinder: **Penzien, Klaus, Dr., Bensheimer Ring 18, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 029 945 B1

BUNDESDRUCKEREI BERLIN

Verfahren zur Herstellung elektrisch leitfähiger Polyene und deren Verwendung in der Elektrotechnik und zur antistatischen Ausrüstung von Kunststoffen

Die Erfindung betrifft ein Verfahren zur Herstellung von elektrisch leitfähigen Polyene und deren Verwendung in der Elektroindustrie zur Herstellung von Sonnenzellen, zur Umwandlung und Fixierung von Strahlung und zur Herstellung elektrischer und magnetischer Schalter sowie zur antistatischen Ausrüstung von Kunststoffen.

Aus Ber. Bunsenges. Phys. Chem. 83, 427 (1979) ist bekannt, daß Polyaromaten, wie Polyphenylene, die Eigenschaft der elektrischen Leitfähigkeit aufweisen.

Da ein Bedarf an organischen Verbindungen mit einer für technische Zwecke ausreichend hohen Leitfähigkeit besteht, war nach neuen leitfähigen organischen Systemen mit genügend hoher Leitfähigkeit zu suchen.

Diese Aufgabe wurde erfindungsgemäß dadurch gelöst, daß man Polyene, die mindestens einmal das Kettenglied der Formel

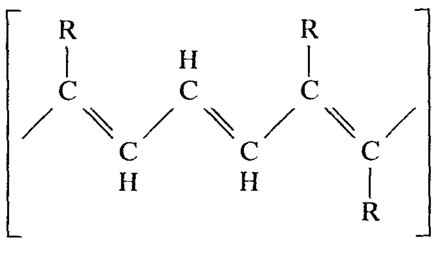

in der R ein Wasserstoffatom oder eine Methylgruppe bedeutet, und insgesamt mindestens sieben aliphatische Doppelbindungen enthalten, unter Ausschluß von Wasserfeuchtigkeit und von Sauerstoff mit 0.03 bis 0,9 Molprozent, bezogen auf das eingesetzte Polyen, einer starken Lewis-Säure mit $pk_s$-Werten von $-10$ bis $+4$ oder eines Alkalimetalls behandelt.

Die nach der Erfindung zu verwendenden Polyene enthalten mindestens einmal das Kettenglied der obengenannten Formel, in der R ein Wasserstoffatom oder eine Methylgruppe bedeutet, und insgesamt mindestens sieben, vorzugsweise 11 bis 20 aliphatische Doppelbindungen. Sie können aliphatische, cycloaliphatische oder aromatische Reste enthalten. Als Substituenten können die Polyene Hydroxylgruppe, Äthergruppen, wie $H_3CO$-Gruppen, Aldehydgruppen, Ketogruppen, Carboxylgruppen, Carbonsäureestergruppen, Chloratome, Sulfongruppen oder Triphenylphosphingruppen enthalten. Die genannten Reste und Substituenten stehen bevorzugt an den Enden der kettenförmigen Polyene.

Beispielsweise seien folgende Polyene genannt:

β-Carotin, Crocetindial, Canthaxanthin, Sarcinen, Sarcinaxanthin, Rhodoxanthin,
1,2,1′,2′-Tetrahydrolycopen,
7,8,11,12-Tetrahydrolycopen,
7,8,7′,8′-Tetrahydrolycopen,
1,2,7,8,11,12-Hexahydrolycopen,
1,2,1′,2′-Tetrahydroneurosporen,
7,8,1′,2′,7′,8′-Hexahydrolycopen, Phytofluen, 1,2-Dihydrophytofluen, Phytoen,
6′,7′-Didehydro-5,6,5′,6′-tetrahydro-β,β-caroten-3,5,6,3′,5′-pentol,
1-Methoxy-1′,2′-dihydro-3′,4′-dehydro-γ-caroten, Anhyhydrorhodovibrin,
3,4-Dihydroanhydrorhodovibrin, Spheroiden, Cryptoxanthin-5,8,5′,8′-diepoxid,
Auroxanthin, Torularhodinaldehyd,
3,4-Dehydrolycopen-16-al,
3-Hydroxy-3′-keto-retrodehydrocaroten,
5,6,5′,6′-Tetrahydrocanthaxanthin, β,α-Caroten-3′,6′-dion,
(3S,5R,3′S,5′R)-3,3′-Dihydroxy-α,α-caroten-6,6′-dion,
7,8,7′,8′-Tetrahydrocapsorubin, Phillipsiaxanthin,
2,2′-Diketospirilloxanthin,
1,1′-Dimethoxy-1,2,1′,2′-tetrahydro-φ,φ-caroten-4,4′-dion,
5-Hydroxy-4′,5′-didehydro-4,5-dihydro-4,5′-retro-β,β-caroten-3,3′-dion,
Capsorubindion, Torularhodin, Torularhodinmethylester,
Methyl-1′-methoxy-4′-oxo-1′,2′-dihydro-χ,ψ-caroten-16(oder 17 oder 18)-oat,
Semi-β-cartoneon, Semi-α-carotenon,
3-Hydroxysemi-β-carotenon,
5,6,5′,6′-Diseco-β,β-carotene-5,6,5′,6′-tetron,
2-(4-Hydroxy-3-methyl-2-butenyl)-β,β-caroten,
7′,8′,11′,12′-Dehydrononaprenoxanthin,
11′,12′-Dehydrononapreoxanthin,

2,2'-Bis(3-methyl-2-butenyl)-ε,ε-caroten,
Deshydroxydecaprenoxanthin, Sarcinaxanthin,
(2R,6S,2'R,6'S)-2,2'-Bis(4-hydroxy-3-methyl-2-butenyl)-γ,γ-caroten,
2-[4-(β-D-Glucopyranosyloxy)-3-methyl-2-butenyl]-2'-(4-hydroxy-3-methyl-2-butenyl)-ε,ε-caroten,
2,2'-Bis[4-(β-D-Glucopyranosyloxy)-3-methyl-2-butenyl]-ε,ε-caroten,
7,8(oder 7',8')-Dihydrodecaprenoxanthin-monoglucosid,
2-(4-Hydroxy-3-methyl-2-butenyl)-2-(3-methyl-2-butenyl)-3',4'-didehydro-1',2'-dihydro-ε,ψ-caroten-1'-ol,
Trisanhydrobacterioruberin.

Die genannten Ausgangsstoffe und ihre Formeln werden z. B. in O. Straub: »Key to Carotenoids«, Birkenhauser Verlag 1976 unter den Nummern 24 bis 32, 96 bis 99, 140 bis 142, 192 bis 194, 205 bis 227 beschrieben.

Als Ausgangsstoffe sind ebenfalls die in Angew. Chemie 89, 198 (1977) beschriebenen, als Makrolide bezeichneten Polyene geeignet, die einen Zuckerrest mit mehreren, vorzugsweise 5 oder 6 Hydroxylgruppen aufweisen. Ebenfalls kommen Polyene ohne Methylverzweigungen, wie der in Angew. Chem. 89, 198 (1977) beschriebene Flexirubindimethylester in Betracht.

Die Polyene werden erfindungsgemäß durch Behandlung mit den Lewis-Säuren oder den Alkalimetallen in die leitfähigen Stoffe übergeführt. Als Lewis-Säuren mit $pk_s$-Werten von $-10$ bis $+4$ eignen sich z. B. die Verbindungen $AsF_5$, $SbF_5$, $HClO_4$, Trifluoressigsäure, $FSO_3H$, $AgClO_4$, $NO^+SbF_6^-$, $NO_2^+SbF_6^-$, $NO^+AsF_6^-$, $NO^+PF_6^-$, $NO_2^+PF_6^-$, $NO^+BF_4^-$, $NO_2^+BF_4^-$, $NO^+ClO_4^-$, $(CF_3)_2SO_4$, 2,4,6-Trinitrophenol, 2,4,6-Trinitrophenylsulfosäure oder 2,4,6-Trinitrophenylcarbonsäure.

Als Alkalimetalle verwendet man z. B. Natrium oder Kalium, die auch in Form von Lösungen, z. B. in Naphthalin oder α-Methylstyrol, eingesetzt werden können.

Die Behandlung der Polyene mit den Lewis-Säuren oder den Alkalimetallen führt man bei Temperaturen von $-70$ bis 150° C, vorzugsweise $-10$ bis 100° C, insbesondere 0 bis 30° C durch. Dabei wendet man die Säuren oder Alkalimetalle, die mit den Polyenen unter Komplexbildung die leitfähigen Verbindungen ergeben, in Mengen von 0,03 bis 0,9, vorzugsweise 0,1 bis 0,5 Mol-%, bezogen auf die Polyene an.

Das Einarbeiten der Zusätze erfolgt unter Ausschluß von Feuchtigkeit (Wasser) sowie Sauerstoff (Luft), es wird daher vorzugsweise unter Edelgas-Argonatmosphäre gearbeitet. Gegebenenfalls werden als Hilfsflüssigkeiten nicht-wäßrige Lösungsmittel, die unter den Verfahrensbedingungen nicht mit den Lewis-Säuren oder den Alkalimetallen reagieren, wie Methylenchlorid, Tetrahydrofuran, Dimethoxyglykol, Nitromethan, Naphthalin oder α-Methylstyrol, eingesetzt. Die meist tieffarbigen elektrisch leitfähigen Polyene werden bei dem erfindungsgemäßen Verfahren in wenigen Sekunden bis Minuten gebildet. Gegebenenfalls verwendete Lösungsmittel werden zweckmäßig bei Temperaturen unter 100° C im Vakuum abgezogen.

Durch das Verfahren der Erfindung werden Polyene mit hoher elektrischer Leitfähigkeit erhalten. Beispielsweise wird die Leitfähigkeit des Canthaxanthin von $1{,}4 \cdot 10^{-10}$ S/cm auf $5{,}1 \cdot 10^{-4}$ S/cm erhöht. Die elektrischen Leitfähigkeitswerte werden in S/cm bei 30° C gemessen, die Messung selbst erfolgt nach der Methode von F. Beck, Berichte Bunsengesellschaft, Physikalische Chemie 68 (1964), Seiten 558 bis 567.

Die erfindungsgemäß hergestellten elektrisch leitfähigen Polyene sind zur antistatischen Ausrüstung von Kunststoffen, zur Herstellung von Sonnenzellen, zur Umwandlung und Fixierung von Strahlung sowie zur Herstellung elektrischer und magnetischer Schalter geeignet.

Die in den folgenden Beispielen genannten Teile sind Gewichtsteile.

## Beispiele 1 bis 10

Man gibt zu 10 Teilen β-Carotin unter Argon bei Raumtemperatur und unter Ausschluß von Wasserfeuchtigkeit und Luftsauerstoff 50 Teile einer 10%igen Lösung von (0,03 Mol-%) $SbF_5$ in Methylenchlorid. Die Komplexbildung tritt sofort ein. Man zieht das Lösungsmittel im Vakuum ab und erhält eine tiefblauschwarze kristalline Verbindung, die beim Messen in einer Leitfähigkeitsmeßzelle eine Leitfähigkeit von $3{,}6 \cdot 10^{-6}$ S/cm aufweist. Die Leitfähigkeit des eingesetzten β-Carotins betrug $3{,}6 \cdot 10^{-10}$ S/cm.

Verfährt man wie in diesem Beispiel 1 beschrieben, wobei man die in der Tabelle genannten Polymere und Zusätze verwendet, so werden Polyene mit den angegebenen Leitfähigkeiten erhalten.

Tabelle

| Beispiel | Polyen Art und Menge | Ausgangsleitfähigkeit bei 25°C [S/cm] | Dotierungsmittel Menge [Teile] und Art | Leitfähigkeit nach dem Dotieren bei 25°C [S/cm] |
|---|---|---|---|---|
| 2 | Crocetindial 10 Teile | $1{,}0 \cdot 10^{-10}$ | 17 $SbF_5$ ca. 0,1 Mol-% | $1{,}24 \cdot 10^{-7}$ |
| 3 | Canthaxanthin 10 Teile | $1{,}4 \cdot 10^{-10}$ | 18 $SbF_5$ ca. 0,1 Mol-% | $5{,}1 \cdot 10^{-4}$ |
| 4 | Canthaxanthin 10 Teile | $1{,}4 \cdot 10^{-10}$ | 19,5 $No^+SbF_6$ ca. 0,1 Mol-% | $7{,}8 \cdot 10^{-4}$ |
| 5 | Canthaxanthin 10 Teile | $1{,}4 \cdot 10^{-10}$ | 21 $NO_2^+SbF_6$ ca. 0,1 Mol-% | $9{,}2 \cdot 10^{-4}$ |
| 6 | Canthaxanthin 10 Teile | $1{,}4 \cdot 10^{-10}$ | 6 K ca. 0,2 Mol-% | $3{,}4 \cdot 10^{-3}$ |
| 7 | Flexirubindimethylester 10 Teile | $3{,}5 \cdot 10^{-10}$ | 20 $SbF_5$ ca. 0,2 Mol-% | $7{,}2 \cdot 10^{-2}$ |
| 8 | Sarcinaxanthin 10 Teile | $2{,}4 \cdot 10^{-10}$ | 22 $CF_3COOH$ ca. 0,25 Mol-% | $3{,}9 \cdot 10^{-3}$ |
| 9 | Sarcinen 10 Teile | $1{,}0 \cdot 10^{-10}$ | 21 $SbF_5$ ca. 0,2 Mol-% | $4{,}8 \cdot 10^{-4}$ |
| 10 | Rhodoxanthin 10 Teile | $1{,}0 \cdot 10^{-10}$ | 15,5 $FSO_3H$ ca. 0,2 Mol-% | $6{,}8 \cdot 10^{-3}$ |

**Patentansprüche**

1. Verfahren zur Herstellung von elektrisch leitfähigen Polyenen, dadurch gekennzeichnet, daß man Polyene, die mindestens einmal das Kettenglied der Formel

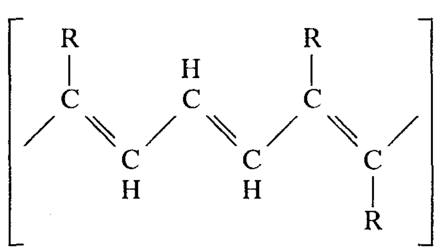

in der R ein Wasserstoffatom oder eine Methylgruppe bedeutet, und insgesamt mindestens sieben aliphatische Doppelbindungen enthalten, unter Ausschluß von Wasserfeuchtigkeit und von Sauerstoff mit 0,03 bis 0,9 Molprozent, bezogen auf das eingesetzte Polyen, einer starken Lewis-Säure mit $pk_s$-Werten von $-10$ bis $+4$ oder eines Alkalimetalls behandelt.

2. Verfahren nach Anspruch 1, dadurch gekennzeichnet, daß man als Lewis-Säure die Verbindung $AsF_5$, $SbF_5$, $HClO_4$, Trifluoressigsäure, $FSO_3H$, $AgClO_4$, $NO^+SbF_6^-$, $NO_2^+SbF_6^-$, $NO^+AsF_6^-$, $NO^+PF_6^-$, $NO_2^+PF_6^-$, $NO^+BF_4^-$, $NO_2^+BF_4^-$, $NO^+ClO_4$, $(CF_3)_2SO_4$, 2,4,6-Trinitrophenol, 2,4,6-Trinitrophenylsulfosäure oder 2,4,6-Trinitrophenylcarbonsäure verwendet.

3. Verfahren nach Anspruch 1, dadurch gekennzeichnet, daß man als Alkalimetall Natrium oder Kalium verwendet.

4. Verwendung der nach Anspruch 1 hergestellten, leitfähigen Polyene in der Elektrotechnik zur Herstellung von Sonnenzellen, zur Umwandlung und Fixierung von Strahlung und zur Herstellung elektrischer und magnetischer Schalter.

5. Verwendung der nach Anspruch 1 hergestellten leitfähigen Polyene zur antistatischen Ausrüstung von Kunststoffen.

## Claims

1. A process for the preparation of an electrically conductive polyene, characterized in that a polyene which contains at least one chain member of the formula

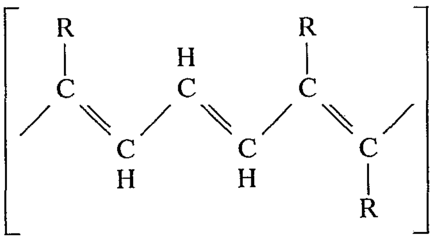

where R is hydrogen or methyl, and which contains a total of not less than seven aliphatic double bonds, is treated, in the absence of moisture and of oxygen, with from 0.03 to 0.9 mole per cent, based on the polyene employed, of a strong Lewis acid having a $pk_a$ of from $-10$ to $+4$ or of an alkali metal.

2. A process as claimed in claim 1, characterized in that $AsF_5$, $SbF_5$, $HClO_4$, trifluoroacetic acid, $FSO_3H$, $AgClO_4$, $NO^+SbF_6^-$, $NO_2^+SbF_6^-$, $NO^+AsF_6^-$, $NO^+PF_6^-$, $NO_2^+PF_6^-$, $NO^+BF_4^-$, $NO_2^+BF_4^-$, $NO^+ClO_4^-$, $(CF_3)_2SO_4$, 2,4,6-trinitrophenol, 2,4,6-trinitrophenylsulfonic acid or 2,4,6-trinitrophenylcarboxylic acid is used as Lewis acid.

3. A process as claimed in claim 1, characterized in that sodium or potassium is used as the alkali metal.

4. Use of an electrically conductive polyene, prepared as claimed in claim 1, in the electrical industry for the produktion of solar cells, for the conversion and fixing of radiation, and for the produktion of electrical and magnetic switches.

5. Use of an electrically conductive polyene, prepared as claimed in claim 1, for the antistatic treatment of plastics.

## Revendications

1. Procédé pour la préparation de polyènes électriquement conducteurs, caractérisé en ce qu'on traite des polyènes, qui contiennent au moins une fois le maillon de formule

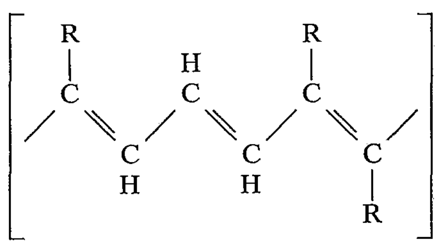

dans laquelle R représente un atome d'hydrogène ou un groupe méthyle et au total au moins sept doubles liaisons aliphatiques, hors la présence d'humidité aqueuse et d'oxygène, par 0,03 à 0,9 mol%, par rapport au polyène utilisé, d'un acide de Lewis fort ayant une valeur de $pk_s$ comprise entre $-10$ et $+4$ ou d'un métal alcalin.

2. Procédé selon la revendication 1, caractérisé en ce qu'on utilise, en tant qu'acide de Lewis, le composé $AsF_5$, $SbF_5$, $HClO_4$, acide trifluoracétique, $FSO_3H$, $AgClO_4$, $NO^+SbF_6^-$, $NO_2^+SbF_6^-$, $NO^+AsF_6^-$, $NO^+PF_6^-$, $NO_2^+PF_6^-$, $NO^+BF_4^-$, $NO_2^+BF_4^-$, $NO^+ClO_4^-$, $(CF_3)_2SO_4$, 2,4,6-trinitrophénol, acide 2,4,6-trinitrophénylsulfonique ou acide 2,4,6-trinitrophénylcarboxylique.

3. Procédé selon la revendication 1, caractérisé en ce qu'on utilise, en tant que métal alcalin, le sodium ou le potassium.

4. Utilisation des polyènes conducteurs, préparés suivant la revendication 1, dans l'électrotechnique pour la fabrication de piles solaires, pour la transformation et la fixation de rayonnements et pour la fabrication de commutateurs électriques et magnétiques.

5. Utilisation des polyènes conducteurs, préparés suivant la revendication 1, pour l'apprêt antistatique de matières synthétiques.